# EUROPÄISCHE PATENTSCHRIFT

(11) **EP 0 432 541 B1**
(45) Veröffentlichungstag und Bekanntmachung des Hinweises auf die Patenterteilung: **13.09.1995**
(21) Anmeldenummer: 90122359.4
(22) Anmeldetag: 23.11.1990
(51) Int. Cl.: C07C 51/235, C07C 55/16, C07C 55/21, C07C 55/02, C07C 45/40

(54) **Verfahren zur Herstellung von alpha-omega-Alkandicarbonsäuren**
Process for preparing alpha-omega alkanoic dicarboxylic acids
Procédé de préparation d'acides alcanoiques alpha-omega dicarboxyliques

(30) Priorität: 14.12.1989 AT 2839/89
(43) Veröffentlichungstag der Anmeldung: 19.06.1991
(73) Patentinhaber: Chemie Linz GmbH, A-4021 Linz (AT)
(72) Erfinder: Schermanz, Karl, Dr., A-8010 Graz (AT); Schöftner, Manfred, A-4020 Linz (AT); Kloimstein, Engelbert, A-4070 Eferding (AT); Schaller, Josef, A-4020 Linz (AT); Perndorfer, Eduard, A-4050 Traun (AT); Reiter, Klaus, A-4020 Linz (AT); Neuhofer, Rudolf, A-4020 Linz (AT)
(74) Vertreter: Kunz, Ekkehard, Dr.

(56) Entgegenhaltungen:
- EP-A- 0 147 593
- EP-A- 0 257 202
- GB-A- 709 450
- GB-A- 2 034 310
- Ullmann's Encyclopedia of Industrial Chemistry vol. A5, 5th ed., 1986 Seite 239; 4.1.1. Aldehyde Oxidation

## Beschreibung

Die Erfindung betrifft ein Verfahren zur Herstellung von alpha, omega-Alkandicarbonsäuren ausgehend von Cycloalkenen und von alpha, omega-Alkandialdehyden.

Alpha, omega-Alkandicarbonsäuren sind wertvolle Ausgangsprodukte in der chemischen Industrie und finden beispielsweise bei der Herstellung von Pharmazeutika, Kosmetika, Schmiermittel u. dgl. Verwendung.

Es sind bereits Verfahren zur Herstellung von alpha, omega-Alkandicarbonsäuren bekannt. Gemäß Ullmann's Encyclopedia of Industrial Chemistry Vol. A5, 5th ed (1986) Seite 239, können organische Säuren aus Olefinen über eine katalytische Hydroformylierungsreaktion mit CO und H₂ erhalten werden, wobei die zunächst entstehenden Aldehyde mit Sauerstoff zu den entsprechenden Säuren oxidiert werden. In EP-A-257.202 ist die Herstellung von 1,12-Dodecandisäure durch katalytische Hydroformylierung von 1,9-Decadien mit CO+H₂ zu 1,12-Dodecandial und anschließende Oxidation mit Sauerstoff beschrieben.
Weiters ist beispielsweise in Chemical Abstracts Vol 96, (1982) 199107 a die Herstellung von alpha, omega-Alkandicarbonsäuren durch oxidative Ringspaltung der entsprechenden Cycloalkene mit Hilfe von Ozon in Gegenwart von Methanol und Schwefelsäure, Oxidation der Peroxidlösung mit H₂O₂ und anschließende Verseifung der entstandenen Dimethylester beschrieben, worauf die entstandene Dicarbonsäure durch Umkristallisieren gereinigt werden muß.

Ferner ist aus Chemical Abstracts Vol 97 (1982) 216903x die Ozonisierung von Cycloocten in Propionsäure zu Acyloxyhydroperoxid, das sich zu omega-Formylcarbonsäure umlagert und anschließende Oxidation dieser Lösung mit H₂O₂ in Gegenwart einer organischen Säure zur Dicarbonsäure bekannt, die ebenfalls umkristallisiert werden muß.

Ein gravierender Nachteil bei diesen beiden Verfahren besteht darin, daß eine peroxidhältige Lösung mit einem Oxidationsmittel versetzt werden muß. Eine Reaktionsmischung bestehend aus einem Peroxid und einem weiteren Oxidationsmittel stellt eine Kombination dar, die sich sehr leicht explosionsartig zersetzen kann. Die Anwendung eines solchen Verfahrens im größeren Maßstab ist daher aus sicherheitstechnischen Aspekten bedenklich.

Es konnte nun ein gefahrloses Verfahren zur Herstellung von alpha, omega-Alkandicarbonsäuren gefunden werden, bei dem ausgehend von Cycloalkenen Alkandicarbonsäuren erhalten werden. Dabei werden die als Ausgangsverbindungen dienenden Cycloalkene mit Ozon behandelt, die bei der Ozonisierung entstandene peroxidhaltige Ozonidlösung katalytisch hydriert und der entstandene Dialdehyd anschließend zur Disäure oxidiert. Die gefährlichen Peroxide bei der Reduktion der peroxidhältigen Lösung werden zerstört und die Oxidation findet in peroxidfreier Lösung statt, wodurch keine Explosionsgefahr mehr besteht. Die Alkandicarbonsäuren fallen dabei in hoher Reinheit an.

Gegenstand der Erfindung ist daher ein Verfahren zur Herstellung von alpha, omega-Alkandicarbonsäuren der allgemeinen Formel I, 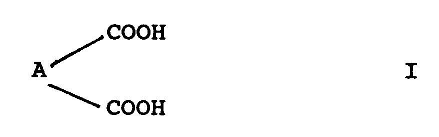
in der A einen Alkylenrest mit 4 - 14 C-Atomen bedeutet, das dadurch gekennzeichnet ist, daß man ein Cycloalken der allgemeinen Formel II, 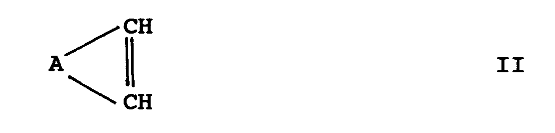
in der A die oben genannte Bedeutung hat, mit Ozon in Gegenwart eines Lösungsmittels umsetzt, die entstandene Peroxidlösung katalytisch hydriert, worauf der entstandene Dialdehyd in Gegenwart eines inerten aprotischen Lösungsmittels zur Disäure der Formel I oxidiert wird.

In den Formeln I und II bedeutet A einen Alkylenrest mit 4 bis 14 C-Atomen, beispielsweise einen Butylen-, Pentylen-, Hexylen-, Heptylen-, Octylen-, Nonylen-, Decylen-, Undecylen-, Dodecylen-, Tridecylen oder Tetradecylenrest. Vorzugsweise bedeutet A einen Pentylen-, einen Hexylen- oder Decylenrest.

Die Ozonisierung und anschließende Hydrierung kann auf übliche Weise durchgeführt werden. Dazu werden die Ausgangsverbindungen in einem geeigneten organischen Lösungsmittel gelöst und mit Ozon behandelt. Die Lösung der Ozonolyseprodukte wird anschließend katalytisch mit Hilfe von Wasserstoff hydriert. Als Katalysatoren eignen sich für Hydrierungen üblicherweise eingesetzte Edelmetallkatalysatoren, die in Form von Pulverkontakten mit Trägermaterialien oder ohne Trägermaterial eingesetzt werden können, beispielsweise Pd-, Pt-, Ru-, Rh-, Ni-Katalysatoren. Als Trägermaterial eignen sich beispielsweise Aktivkohle gegebenenfalls mit einem Erdalkalimetallcarbonat, Aluminiumoxide, Silikagel und Kieselgur. Vorzugsweise wird Pt oder Pd/C mit CaCO₃ als Katalysator eingesetzt.

Vorzugsweise werden Ozonisierung und anschließende Hydrierung nach dem in EP-A-0 147 593 beschriebenen Verfahren durchgeführt. Dabei wird zur Durchführung der Reaktion das Cycloalken in einem organischen Lösungsmittel, in dem es gut löslich ist, beispielsweise in einem niedrigen aliphatischen Alkohol, vorzugsweise in Methanol gelöst und mit der äquivalenten Menge Ozon behandelt. Die Ozonisierung wird bei Temperaturen von etwa -30 bis 0°C, vorzugsweise etwa -20 bis 0°C durchgeführt.

Die an die Ozonisierung anschließende katalytische Hydrierung der Ozonolyseprodukte wird in verdünnter Lösung durchgeführt, wobei vorzugsweise während der Hydrierung ein kontrollierter Peroxidgehalt von höchstens 0,1 mol/l eingehalten wird. Dazu wird eine Suspension des Katalysators im Lösungsmittel und Wasserstoff vorgelegt und die Ozonolyselösung kontinuierlich eingespeist. Durch den durch diese Maßnahme niedrig gehaltenen Peroxidgehalt des Reaktionsmediums wird eine Vergiftung und ein Aktivitätsverlust des Katalysators verhindert.

Die Hydrierung erfolgt unter praktisch drucklosen Bedingungen, das sind Drücke von 1 bis 3 bar, die üblicherweise angewendet werden, um das Eindringen von Luft in den Hydrierreaktor zu verhindern.
Die Reaktionstemperatur bei der Hydrierung beträgt etwa 20 - 40°C. Der pH-Wert wird während der Hydrierung in einem Bereich von 2 - 7 gehalten. Da während der Hydrierung geringe Mengen an sauren Nebenprodukten entstehen können, kann der pH-Wert gegebenenfalls durch Zugabe einer Base, vorzugsweise verdünnter Natronlauge oder Kalilauge im gewünschten Bereich gehalten werden.
Der Katalysator wird anschließend abfiltriert und das Lösungsmittel entfernt. Der Rückstand wird in einem für den anschließenden Oxidationsschritt geeigneten inerten Lösungsmittel gelöst und mit Hilfe eines Oxidationsmittels oxidiert.
Als Lösungsmittel kommen unter Reaktionsbedingungen inerte aprotische, organische Lösungsmittel, beispielsweise aromatische Kohlenwasserstoffe wie Benzol, Toluol, Chlorbenzol, Dichlorbenzol und dergleichen in Betracht. Als Oxidationsmittel können übliche Oxidationsmittel wie Peroxide z. B. H₂O₂, Sauerstoff in Verbindung mit Metallsalzen als Katalysator und dgl. eingesetzt werden. Es hat sich jedoch herausgestellt, daß die Oxidation unter Verwendung von Luft oder Sauerstoff, bevorzugt von Sauerstoff als Oxidationsmittel ohne Katalysatoren, wie Metallsalze einsetzen zu müssen abläuft, wobei die Dicarbonsäuren dennoch einheitlich in hoher Reinheit und ausgezeichneten Ausbeuten anfallen. Als Oxidationsmittel wird dementsprechend bevorzugt Sauerstoff oder Luft, ganz bevorzugt Sauerstoff eingesetzt.

Die Oxidation wird vorzugsweise unter Druck durchgeführt, wobei Drücke von 3 - 10 bar bevorzugt sind. Die Reaktion wird bei Temperaturen von 50 - 100°C, vorzugsweise bei 70 - 90°C durchgeführt. Ein Verfahren zur Oxidation einer alpha, omega-Dicarbonsäure ausgehend von alpha, omega-Dialdehyden in einem inerten, aprotischen, organischen Lösungsmittel unter Verwendung von Luft oder Sauerstoff als Oxidationsmittel unter Druck bei Temperaturen von 50 bis 100°C ist neu und ebenfalls Gegenstand der Erfindung.

Nach beendeter Oxidation wird die Reaktionslösung abgekühlt, auf ein bestimmtes Volumen eingeengt oder das Lösungsmittel entfernt, wobei die Dicarbonsäure in kristalliner Form anfällt.

Nach dem erfindungsgemäßen Verfahren werden alpha, omega-Dicarbonsäuren in hoher Reinheit und mit ausgezeichneten Ausbeuten erhalten. In der Regel werden Ausbeuten von 65 - 85 %, nach Aufarbeitung der Mutterlauge von 75 - 95 % erreicht. Die Dicarbonsäuren werden mit mindestens 95 %iger Reinheit erhalten, wodurch üblicherweise eine weitere Reinigung des Endproduktes nicht nötig ist.

### Beispiel 1: Dodecandisäure

### Dodecandial:

130,1 g (1 mol) Cyclododecen (Reinheit 95 %) wurden in 1500 ml Methanol gelöst, auf -20°C gekühlt und ein O₂/O₃-Gemisch, das 4 Gew.% Ozon enthält eingeleitet, bis 1 mol Ozon in die Lösung eingebracht worden sind.
Anschließend wurden in einem Hydrierreaktor, in dem 5 g Pd/C mit CaCO₃ (Lindlar Katalysator) vorgelegt wurden und der mit Wasserstoff gefüllt wurde, die erhaltene Ozonisierungslösung über ein Dosiergefäß kontinuierlich eingespeist, daß der Peroxidgehalt 0,02 mol/l nicht übersteigt. Unter kräftigen Rühren und Wasserstoffzugabe wurde bis zur negativen Peroxidprobe hydriert.
Die erhaltene Hydrierlösung enthielt 72,96 % der Theorie Dodecandial (GC).

### Dodecandisäure:

Aus der Hydrierlösung wurde das Lösungsmittel abgedampft und der Rückstand in 1000 ml Chlorbenzol gelöst. Die Lösung wurde in einem Autoklaven mit Sauerstoff bei einem Druck von 5 bar unter Rühren versetzt und auf 80°C erwärmt. Nach beendeter Reaktion wurde der Autoklav entspannt, die Lösung auf Raumtemperatur abgekühlt und die Lösungen auf etwa 300 ml eingeengt.
Der sich bildende kristalline Niederschlag wurde abfiltriert und mit Wasser gewaschen. Es wurden 151 g (0,655 mol) Dodecandisäure (65 % bezogen auf Cyclododecen) mit einer Reinheit von 96 % erhalten.

### Beispiel 2:

### Oktandial:

110,2 g (1 mol) Cycloocten (Reinheit 95 %) wurden in 1500 ml Methanol gelöst auf -20°C gekühlt und ein O₂/O₃-Gemisch, das 4 Gew.% Ozon enthält, eingeleitet, bis 1 mol Ozon in die Lösung eingebracht worden sind.

Anschließend wurden in einem Hydrierreaktor, in dem 5,0 g Lindlar-Katalysator (Pd/C mit CaCO₃) vorgelegt wurden, und der mit Wasserstoff gefüllt wurde, die erhaltene Ozonisierungslösung über ein Dosiergerät kontinuierlich eingespeist, daß der Peroxidgehalt 0,02 mol/l nicht übersteigt. Unter kräftigem Rühren und Wasserstoffzugabe wurde bis zur negativen Peroxidprobe hydriert. Der pH-Wert fiel von 7 auf 5,4. Die erhaltene Hydrierlösung enthielt 78,75% der Theorie Octandial.

### Octandisäure:

Aus der Hydrierlösung wurde das Lösungsmittel entfernt und der Rückstand in 1000 ml Chlorbenzol gelöst. Diese Lösung wurde in einem Autoklaven mit Sauerstoff bei einem Druck von 5 bar unter Rühren versetzt und die Temperatur auf etwa 80°C gehalten. Nach beendeter Reaktion wurde der Autoklav entspannt, die Lösung auf Raumtemperatur gekühlt und das Lösungsmittel bis auf etwa 300 ml entfernt. Der sich bei Kühlung auf etwa 7°C bildende Niederschlag wurde abfiltriert und mit Wasser gewaschen.
Es wurden 116,2 g (0,702 mol) Octandisäure (70,2 %) bezogen auf eingesetztes 95 %iges Cycloocten mit einer Reinheit von 98,5 % erhalten.

## Patentansprüche

1. Verfahren zur Herstellung von alpha, omega-Alkandicarbonsäuren der allgemeinen Formel I 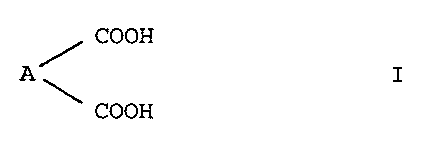 in der A einen Alkylenrest mit 4 - 14 C-Atomen bedeutet, dadurch gekennzeichnet, daß man ein Cycloalken der allgemeinen Formel II 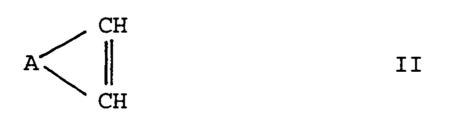 in der A die oben genannte Bedeutung hat, mit Ozon in Gegenwart eines inerten Lösungsmittels umsetzt, die entstandene Peroxidlösung katalytisch hydriert, worauf der entstandene Dialdehyd in Gegenwart eines inerten aprotischen Lösungsmittels zur Disäure der Formel I oxidiert wird.

2. Verfahren nach Anspruch 1, dadurch gekennzeichnet, daß als Ausgangsprodukt Cyclohepten, Cycloocten oder Cyclododecen eingesetzt wird.

3. Verfahren nach einem der Ansprüche 1 oder 2, dadurch gekennzeichnet, daß die Umsetzung mit Ozon in Gegenwart von Methanol durchgeführt wird.

4. Verfahren nach Anspruch 3, dadurch gekennzeichnet, daß die Umsetzung mit der äquivalenten Menge Ozon durchgeführt wird.

5. Verfahren nach einem der Ansprüche 1 oder 2, dadurch gekennzeichnet, daß während der Hydrierung der Ozonolyseprodukte ein Peroxidgehalt von höchstens 0,1 mol/l eingehalten wird.

6. Verfahren nach Anspruch 5, dadurch gekennzeichnet, daß der pH-Wert während der katalytischen Hydrierung im Bereich von 2 - 7 gehalten wird.

7. Verfahren nach einem der Ansprüche 5 oder 6, dadurch gekennzeichnet, daß als Katalysator Pd auf Aktivkohle mit CaCO₃, oder Pt eingesetzt wird.

8. Verfahren nach einem der Ansprüche 1 oder 2, dadurch gekennzeichnet, daß im an die Hydrierung anschließenden Oxidationsschritt Sauerstoff oder Luft als Oxidationsmittel verwendet wird.

9. Verfahren nach Anspruch 8, dadurch gekennzeichnet, daß die Oxidation bei einem Druck von 3 - 10 bar und bei einer Temperatur von 70 bis 90°C durchgeführt wird.

10. Verfahren zur Herstellung einer alpha, omega-Alkandicarbonsäure der allgemeinen Formel I nach Anspruch 1, ausgehend vom entsprechenden alpha, omega-Alkandialdehyd, dadurch gekennzeichnet, daß ein alpha, omega-Alkandialdehyd in einem inerten, aprotischen, organischen Lösungsmittel gelöst wird und mit Luft oder Sauerstoff als Oxidationsmittel unter Druck bei Temperaturen von 50 bis 100°C zu der entsprechenden alpha, omega-Alkandicarbonsäure umgesetzt und wie üblich isoliert wird.

## Claims

1. Process for the preparation of alpha, omega-alkanedicarboxylic acids of the general formula I 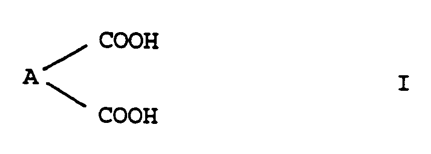 in which A is an alkylene radical having 4 - 14 C atoms, characterised in that a cycloalkene of the general formula II 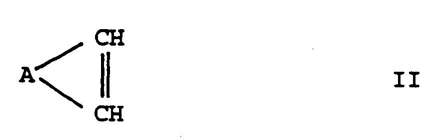 in which A has the abovementioned meaning is reacted with ozone in the presence of an inert solvent, the peroxide solution formed is hydrogenated catalytically, followed by oxidation of the dialdehyde formed to the diacid of the formula I in the presence of an inert aprotic solvent.

2. Process according to Claim 1, characterised in that cycloheptene, cyclooctene or cyclododecene is used as starting material.

3. Process according to one of Claims 1 or 2, characterised in that the reaction with ozone is carried out in the presence of methanol.

4. Process according to Claim 3, characterised in that the reaction is carried out with the equivalent amount of ozone.

5. Process according to one of Claims 1 or 2, characterised in that during the hydrogenation of the ozonolysis products a peroxide content of at most 0.1 mol/l is maintained.

6. Process according to Claim 5, characterised in that the pH during the catalytic hydrogenation is maintained in the range of 2 - 7.

7. Process according to one of Claims 5 or 6, characterised in that Pd on activated carbon in combination with CaCO₃ or Pt is used as the catalyst.

8. Process according to one of Claims 1 or 2, characterised in that oxygen or air is used as the oxidising agent in the oxidation step which follows the hydrogenation.

9. Process according to Claim 8, characterised in that the oxidation is carried out at a pressure of 3 - 10 bar and at a temperature of 70 to 90°C.

10. Process for the preparation of an alpha, omega-alkanedicarboxylic acid of the general formula I according to Claim 1, starting from the corresponding alpha, omega-alkanedialdehyde, characterised in that an alpha, omega-alkanedialdehyde is dissolved in an inert, aprotic, organic solvent and reacted with air or oxygen as the oxidising agent under pressure at temperatures of 50 to 100°C to the corresponding alpha, omega-alkanedicarboxylic acid and isolated as usual.

## Revendications

1. Procédé de préparation d'acides alpha,oméga-alcanedicarboxyliques répondant à la formule générale I : 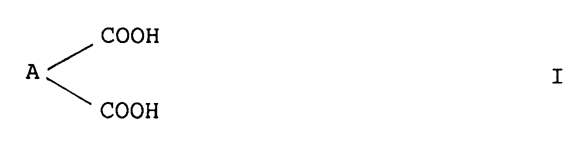 dans laquelle A signifie un reste alkylène ayant de 4 à 14 atomes de carbone,
caractérisé en ce qu'on fait réagir un cycloalkène répondant à la formule générale II : 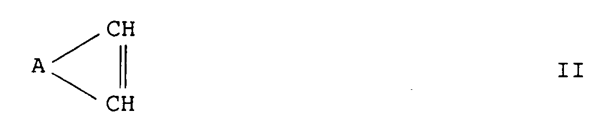 dans laquelle A a la signification indiquée ci-dessus, avec de l'ozone en présence d'un solvant inerte, on soumet la solution peroxydique formée à une hydrogénation catalytique et ensuite, on oxyde le dialdéhyde formé en un diacide de formule I en présence d'un solvant aprotique inerte.

2. Procédé selon la revendication 1, caractérisé en ce qu'on utilise, comme produit de départ, le cycloheptène, le cyclooctène ou le cyclododécène.

3. Procédé selon la revendication 1 ou 2, caractérisé en ce qu'on effectue la réaction avec l'ozone en présence de méthanol.

4. Procédé selon la revendication 3, caractérisé en ce qu'on effectue la réaction avec une quantité équivalente d'ozone.

5. Procédé selon la revendication 1 ou 2, caractérisé en ce que, pendant l'hydrogénation des produits de l'ozonolyse, on limite la teneur en peroxydes à la valeur maximale de 0,1 mole/l.

6. Procédé selon la revendication 5, caractérisé en ce que, pendant l'hydrogénation catalytique, on maintient le pH à une valeur comprise entre 2 et 7.

7. Procédé selon la revendication 5 ou 6, caractérisé en ce qu'on utilise, comme catalyseur, Pd sur du charbon actif avec CaCO₃ ou Pt.

8. Procédé selon la revendication 1 ou 2, caractérisé en ce que, pendant l'opération d'oxydation faisant suite à l'hydrogénation, on utilise de l'oxygène ou de l'air comme oxydant.

9. Procédé selon la revendication 8, caractérisé en ce qu'on effectue l'oxydation à une pression de 3 à 10 bars et à une température de 70 à 90°C.

10. Procédé de préparation d'un acide alpha,oméga-alcane-dicarboxylique répondant à la formule générale I en partant de l'aldéhyde alpha,oméga-alcane-dicarboxylique correspondant, caractérisé en ce qu'on dissout un aldéhyde alpha,oméga-alcane-dicarboxylique dans un solvant organique inerte aprotique, on le transforme en l'acide alpha,oméga-alcane-dicarboxylique correspondant, avec de l'air ou de l'oxygène, comme oxydant, sous pression, à des températures de 50 à 100°C, et on l'isole de façon usuelle.
